# EUROPÄISCHE PATENTSCHRIFT

(11) **EP 1 631 552 B1**
(45) Veröffentlichungstag und Bekanntmachung des Hinweises auf die Patenterteilung: **03.07.2013**
(21) Anmeldenummer: 04731903.3
(22) Anmeldetag: 10.05.2004
(51) Int. Cl.: C07D 233/50, C07D 265/08, C07D 235/30, C07D 409/12, C07D 495/04, C07D 413/12, C07D 277/18, C07D 265/18

(54) **VERFAHREN ZUR SYNTHESE HETEROCYCLISCHER VERBINDUNGEN AUS THIOHARNSTOFFDERIVATEN**
METHOD FOR SYNTHESISING HETEROCYCLIC COMPOUNDS FROM THIOUREA DERIVATES
PROCEDE DE SYNTHESE DE COMPOSES HETEROCYCLIQUES A PARTIR DE DERIVES DE THIOURÉE

(30) Priorität: 22.05.2003 DE 10323701
(43) Veröffentlichungstag der Anmeldung: 08.03.2006
(73) Patentinhaber: Sanofi-Aventis Deutschland GmbH, 65929 Frankfurt am Main (DE)
(72) Erfinder: HEINELT, Uwe, 65187 Wiesbaden (DE); LANG, Hans-Jochen, 65719 Hofheim (DE)
(74) Vertreter: Michalski Hüttermann & Partner Patentanwälte
(86) Internationale Anmeldenummer: PCT/EP2004/004955
(87) Internationale Veröffentlichungsnummer: WO 2004/103976

(56) Entgegenhaltungen:
- KIM T H ET AL: "A mild cyclodesulfurization of N-(2-hydroxyethyl)-N'-phenylthioureas to 2-phenylamino-2-oxazolines using TsCl/NaOH" TETRAHEDRON, ELSEVIER SCIENCE PUBLISHERS, AMSTERDAM, NL, Bd. 57, Nr. 33, 13. August 2001 (2001-08-13), Seiten 7137-7141, XP004298061 ISSN: 0040-4020
- KIM T H ET AL: "One-pot synthesis of 2-phenylaminothiazolines from N-(2-hydroxyethyl)-N'-phenylthioureas" TETRAHEDRON LETTERS, ELSEVIER SCIENCE PUBLISHERS, AMSTERDAM, NL, Bd. 40, Nr. 47, 19. November 1999 (1999-11-19), Seiten 8201-8204, XP004188408 ISSN: 0040-4039 -& "Corrigendum to "One-pot synthesis of 2-phenylaminothiazolines from N-(2-hydroxyethyl)-N'-phenylthioureas"" TETRAHEDRON LETTERS, Bd. 42, 2001, Seite 2413, XP002302483
- KIM T H ET AL: "N-Acyl-4,5-dihydro-4,4-dimethyl-N-methyl- 2-thiazolamine as a chemoselective acylating agent" TETRAHEDRON LETTERS, ELSEVIER SCIENCE PUBLISHERS, AMSTERDAM, NL, Bd. 43, Nr. 52, 23. Dezember 2002 (2002-12-23), Seiten 9553-9557, XP004395733 ISSN: 0040-4039
- GOERDELER J ET AL: "Über Thioacyl-isocyanate, V. Substituierte Thiazolin-dione-(4.5) und ihre thermische Spaltung in Isocyanate und Senföle" CHEMISCHE BERICHTE, VERLAG CHEMIE GMBH. WEINHEIM, DE, Bd. 99, Nr. 11, 10. November 1966 (1966-11-10), Seiten 3572-3581, XP002276496 ISSN: 0009-2940
- MATSUO M ET AL: "New 2-aryliminoimidazilidines. I. Synthesis and antihypertensive properties of 2-(2-phenoxyphenylimino)imidazolidines and related compounds" CHEMICAL AND PHARMACEUTICAL BULLETIN, PHARMACEUTICAL SOCIETY OF JAPAN. TOKYO, JP, Bd. 33, Nr. 10, Oktober 1985 (1985-10), Seiten 4409-4421, XP002276472 ISSN: 0009-2363
- SETH P P ET AL: "Efficient solution phase synthesis of 2-(N-acyl)-aminobenzimidazoles" TETRAHEDRON LETTERS, ELSEVIER SCIENCE PUBLISHERS, AMSTERDAM, NL, Bd. 43, Nr. 41, 7. Oktober 2002 (2002-10-07), Seiten 7303-7306, XP004389280 ISSN: 0040-4039
- OMAR A-M M E: "The cyclodesulfurization of thio compounds; VII. A new facile synthesis of N(alpha)-substituted benzimidazoles", SYNTHESIS, GEORG THIEME VERLAG, STUTTGART, DE, no. 1, 1 January 1974 (1974-01-01), pages 41-42, XP002192342, ISSN: 0039-7881
- OMAR A ET AL: "The Cyclodesulfurization of Thio Compounds; XVI. Dicyclohexylcarbodiimide as an Efficient Cyclodesulfurizing Agent in the Synthesis of Heterocyclic Compounds from Various Thio Compounds", SYNTHESIS, GEORG THIEME VERLAG, STUTTGART, DE, 1 January 1977 (1977-01-01), pages 864-865, XP002412804, ISSN: 0039-7881

## Beschreibung

Gegenstand der Erfindung ist das in Schema 2 dargestellte Verfahren zur Synthese von heterocyclischen Verbindungen der Formel Ia.

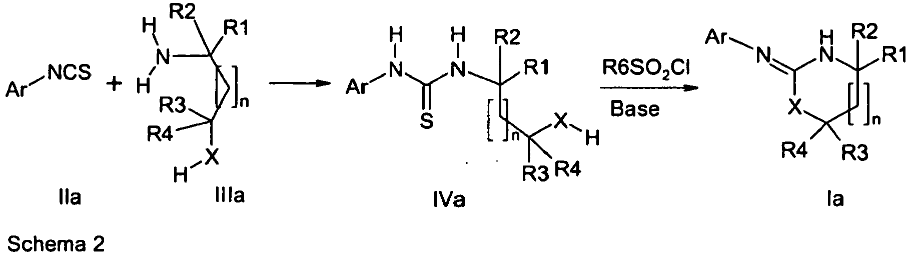

Dabei wird zunächst das Isothiocyanat der Formel IIa mit dem primären Amin der Formel IIIa zum Thioharnstoff der Formel IVa umgesetzt. Anschließend wird der Thioharnstoff der Formel IVa mit einer Base und einem Sulfonsäurechlorid in den Heterocyclus der Formel Ia überführt.

Der Aufbau heterocyclischer Grundgerüste ist einer der wichtigsten Syntheseschritte in der organischen Chemie. Die resultierenden heterocyclischen Verbindungen sind unter anderem von großer Bedeutung als Zwischenprodukte in der Synthese von Arzneimittelwirkstoffen und Pflanzenschutzmittelwirkstoffen oder auch direkt als solche Wirkstoffe. Weiterhin stellt die heutzutage bei der Herstellung von Screening-Substanzen besonders wichtige rasche Synthese vieler, zum Teil strukturell recht diverser Analoga an die Syntheseplanung hohe Anforderungen. Zentrale Bausteine, die den unmittelbaren Zugang zu einer Vielzahl diverser Heterocyclen unter ähnlichen oder im Idealfall identischen Reaktionsbedingungen erlauben, sind daher besonders wertvoll und vor allem für die Roboter-gestützten Synthesen von großer Bedeutung.

Die Heterocyclensynthese ausgehend von Thioharnstoffen ist seit langem bekannt. Allerdings haben die Methoden Limitierungen in der Substratauswahl oder Nachteile in Reaktionsführung, Aufarbeitung, Nebenproduktabtrennung oder im Preis der Reagenzien. So lassen sich 1-(2-Hydroxy-ethyl)-3-aryl-thioharnstoffe durch Schwermetall-Derivate wie Quecksilber(II)oxid oder Bleioxid zu Oxazolidin-2-yliden-aryl-aminen cyclisieren (Jen, et al., J. Med. Chem. 1975 (18), 90). Säurekatalyse derselben Edukte liefert die entsprechenden Aryl-thiazolidin-2-yliden-amine (Jen, et al., J. Med. Chem. 1975 (18), 90). Die Verwendung der Schwermetalle ist jedoch nachteilig, da diese im Produkt, sei es auch nur in Spuren, unerwünscht sind. Die säurekatalysierte Umsetzung zum Thiazolidin wiederum verläuft nur bei erhöhter Temperatur und in Gegenwart hoher Säurekonzentrationen zufriedenstellend. Diese drastischen Bedingungen werden von einigen Funktionalitäten wie Estern, Nitrilen oder Ketalen nicht toleriert.

Synthesen ausgehend von 1-(2-Amino-ethyl)-3-aryl-thioharnstoffen zu Imidazolidin-2-yliden-aryl-Derivaten gelingen in Gegenwart von Methyliodid (Synthesis 1974, 41-42) oder Carbodiimid-Derivaten (Synthesis 1977, 864). Nachteilig bei Methyliodid ist die auftretende Konkurrenz-Reaktion an anderen nukleophilen Zentren im Molekül und sein Gefahrenpotenzial bei unbeabsichtigter Freisetzung. Bei Carbodiimid-Derivaten ist die Abtrennung der gebildeten Harnstoffe häufig problematisch und zeitintensiv. Neuere Carbodiimid-Derivate wie EDC (N'-(3-Dimethylaminopropyl)-N-ethylcarbodiimid Hydrochlorid) oder festphasengebundenes DCC-(Dicyclohexylcarbodiimid) sind wiederum in größerer Menge eingesetzt sehr kostenintensiv.

Die Synthese-Methode der vorliegenden Erfindung führt ausgehend von Isothiocyanaten und Aminoalkoholen, Aminomercaptanen und Diaminen über die intermediär gebildeten Thioharnstoffe zu den gewünschten Heterocyclen variabler Ringgröße, indem man die intermediären Derivate in Gegenwart eines Sulfonsäurechlorids und einer Base cyclisiert. Diese Reagenzien sind kostengünstig, gut handhabbar, erfordern keine drastischen Reaktionsbedingungen und ihre Folgeprodukte sind durch einfache Waschungen leicht zu beseitigen, so dass dieses Syntheseverfahren beispielsweise für Umsetzungen im Gramm- und Kilogrammmaßstab geeignet ist. Es ist aber auch für Parallel- und Robotersynthesen, die meist im Milligrammmaßstab durchgeführt werden, insbesondere wegen der einfachen Reaktionsführung anwendbar. Besonders interessant für diese in der Regel im kleineren Maßstab angewandten Synthesemethoden ist die Verwendung von polymergebundenem Sulfonsäurechlorid, das die Isolierung der Reaktionsprodukte durch apparativ einfache Filtrations- und Verdampfungsschritte ermöglicht.

In der Literatur findet sich eine ähnliche Verfahrensmethode ganz speziell für die Umsetzung von Phenyl- oder Methylisothiocyanaten mit 2-Hydroxyethylaminen zu Oxazolidin- oder Thiazolidin-2-yliden-aminen (Tetrahedron Letters 40 (1999), 8201; Tetrahedron 57 (2001), 7137; Bull. Korean Chem. Soc. 2002 (23), 19).

Überraschend konnte nun gezeigt werden, dass unter diesen Bedingungen nicht nur Fünfringe wie Oxazolidine oder Thiazolidine gebildet werden können, sondern dass Ringgröße und Substitutionsgrad viel flexibler sind und die Synthesemethode nicht auf die Verwendung von 2-Hydroxyethylaminen beschränkt ist. Durch die Beschränkung auf Thioharnstoff-Intermediate, die mindestens einen Arylsubstituenten an einem der Thioharnstoffstickstoffe tragen, erfolgt dabei der Ringschluss sehr selektiv und liefert unter Verlust des Thioharnstoffschwefels im Allgemeinen nur ein Cyclisierungsprodukt.

Die vorliegende Erfindung betrifft somit ein Verfahren zur Herstellung von Heterocyclen der Formel la, 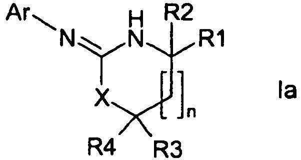 worin bedeuten:
- X: NR5, wobei R5 Wasserstoff oder (C1-C4)-Alkyl entspricht;
- n: Null, 1, 2 oder 3;
- Ar: Phenyl, Naphthyl oder Heteroaryl, die mit 1, 2, 3, 4 oder 5 Resten R11 substituiert sein können,
wobei R11 unabhängig voneinander aus der Gruppe (C1-C4)-Alkyl, F, Cl, Br, I, CN, NO₂, OH, O(C1-C4)-Alkyl, COO(C1-C4)-Alkyl gewählt ist und wobei die Wasserstoffatome der Alkylreste ganz oder teilweise durch Fluoratome ersetzt sein können;
- R1, R2, R3 und R4: unabhängig voneinander Wasserstoff, F oder (C1-C4)-Alkyl, wobei die Wasserstoffatome der Alkylreste ganz oder teilweise durch Fluoratome ersetzt sein können;
oder
- R1 und R3: zusammen eine Bindung,
und
- R2 und R4: mit den beiden Kohlenstoffatomen, an die sie gebunden sind, einen aromatischen Kohlenstoff-Sechsring, in dem ein oder zwei Kohlenstoffatome durch Stickstoff ersetzt sein können, wobei der aromatische Sechsring mit 1, 2, 3 oder 4 Resten R7 substituiert sein kann,
wobei R7 unabhängig voneinander aus der Gruppe (C1-C4)-Alkyl, F, Cl, Br, I, CN, NO₂, OH, O(C1-C4)-Alkyl, COO(C1-C4)-Alkyl gewählt ist und wobei die Wasserstoffatome der Alkylreste ganz oder teilweise durch Fluoratome ersetzt sein können,
wobei n = 0 ist;
oder
- R1 und R3: unabhängig voneinander Wasserstoff oder (C1-C4)-Alkyl
und
- R2 und R4: mit den beiden Kohlenstoffatomen, an die sie gebunden sind, einen gesättigten 5-, 6-, 7- oder 8-gliedrigen Kohlenstoffring, in dem ein oder zwei Kohlenstoffatome durch O, S, NH und N(C1-C4)-Alkyl ersetzt sein können und der mit 1, 2, 3, 4, 5 oder 6 Resten R8 substituiert sein kann,
wobei R8 unabhängig voneinander aus der Gruppe (C1-C4)-Alkyl, O(C1-C4)-Alkyl, COO(C1-C4)-Alkyl gewählt ist, wobei die Wasserstoffatome der Alkylreste ganz oder teilweise durch Fluoratome ersetzt sein können, wobei n = 0 ist;
und deren Tautomere und deren Salze,
dadurch gekennzeichnet, dass, wie in Schema 2 dargestellt, 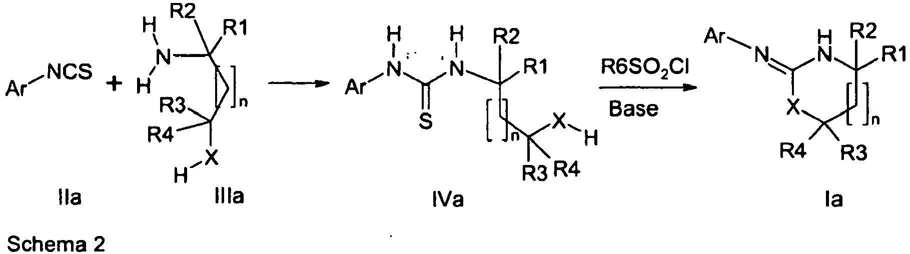
a) ein aromatisches Isothiocyanat der Formel IIa mit einem primären Amin der Formel IIIa zum Thioharnstoff der Formel IVa umgesetzt wird, und
b) der Thioharnstoff der Formel IVa mit einem Sulfonsäurechlorid R6SO₂Cl in Gegenwart einer Base in die Verbindung der Formel Ia überführt wird,
wobei in den Verbindungen der Formeln IIa, IIIa und IVa
- Ar, X, n und R1 bis R4: definiert sind wie in Formel Ia und
- R6: Phenyl ist, welches unsubstituiert ist oder substituiert ist mit Methyl, Trifluormethyl, F, Cl oder Br.

Verfahrensschritt a) kann kontinuierlich oder diskontinuierlich erfolgen. Die Umsetzung des Isothiocyanats der Formel IIa mit dem primären Amin der Formel IIIa kann in Gegenwart eines Lösungsmittels bzw. Verdünnungsmittels durchgeführt werden oder ohne Zusatz eines Lösungsmittels. Bevorzugt wird sie in Gegenwart eines Lösungsmittels durchgeführt. Es können verschiedene Lösungsmittel eingesetzt werden, zum Beispiel aliphatische oder aromatische Kohlenwasserstoffe, chlorierte Kohlenwasserstoffe wie zum Beispiel Methylenchlorid, Ester wie zum Beispiel Ethylacetat, Alkohole oder Ether. Bevorzugt werden Ether als Lösungsmittel eingesetzt, zum Beispiel Tetrahydrofuran, Dioxan oder Ethylenglykolether wie Ethylenglykoldimethylether, insbesondere, wenn die Gesamtreaktion als Eintopfreaktion durchgeführt wird. Es können auch Mischungen aus zwei oder mehr Lösungsmitteln eingesetzt werden. Die Temperatur für die Umsetzung im Verfahrenschritt a) beträgt vorzugsweise 0°C bis zu m Siedepunkt des verwendeten Lösungsmittels, besonders bevorzugt 20°C bis 60°C, zum Beispiel ungefähr Raumtemperatur. Das Isothiocyanat der Formel IIa und das primäre Amin der Formel IIIa werden beispielsweise im molaren Verhältnis 1 : 1,1 bis 1 : 0,9 eingesetzt, bevorzugt in etwa äquimolaren Mengen. Es kann jedoch auch ein Überschuss des Amins der Formel IIIa eingesetzt werden, zum Beispiel wenn X NR5 ist, um Nebenreaktionen zu verhindern.

Verfahrensschritt b) kann kontinuierlich oder diskontinuierlich erfolgen. Im allgemeinen wird die Überführung des Thioharnstoffs der Formel IVa in die Verbindung der Formel Ia in Gegenwart eines Lösungsmittel bzw. Verdünnungsmittels durchgeführt. Es können verschiedene Lösungsmittel eingesetzt werden, zum Beispiel Ester oder Ether, bevorzugt Ether wie zum Beispiel Tetrahydrofuran, Dioxan oder Ethylenglykolether wie Ethylenglykoldimethylether. Als Lösungsmittel kann beispielsweise auch Wasser verwendet werden. Es können auch Mischungen aus zwei oder mehr Lösungsmitteln eingesetzt werden, zum Beispiel Mischungen aus Wasser und einem oder mehreren organischen Lösungsmitteln, beispielsweise Mischungen aus Wasser und einem der genannten Ether. Die Umsetzung kann als Einphasenreaktion oder als Zweiphasenreaktion ablaufen. Die Temperatur für die Umsetzung im Verfahrenschritt b) beträgt vorzugsweise 0°C bis 35 °C, besonders be vorzugt ungefähr Raumtemperatur. Der Thioharnstoff der Formel IVa und das Sulfonylchlorid R6SO₂Cl werden beispielsweise im molaren Verhältnis 1 : 1,4 bis 1 : 0,9 eingesetzt, bevorzugt im Verhältnis 1:1 bis 1 : 1,2, beispielsweise etwa im Verhältnis 1 : 1,1. Bei Einsatz eines polymergebundenen Sulfonylchlorids kann das Verhältnis bei 1 : 1 bis 1 : 4, bevorzugt 1 : 1,5 bis 2,5 liegen. Das molare Verhältnis des Thioharnstoffs der Formel IV zur Base beträgt im Verfahrensschritt b) beispielsweise 1 : 4 bis 1 : 1, vorzugsweise im Verhältnis 1 : 3 bis 1 : 2, beispielsweise etwa im Verhältnis 1 : 2,5. Als Base können im Verfahrensschritt b) verschiedene anorganische oder organische Verbindungen eingesetzt werden, zum Beispiel basische Alkalimetallverbindungen oder Erdalkalimetallverbindungen, insbesondere die Metallhydroxide, oder Amine oder Ammoniumhydroxide. Bevorzugt werden basische Natriumverbindungen oder Kaliumverbindungen als Base verwendet, zum Beispiel Natriumhydroxid, Kaliumhydroxid, Natriumcarbonat oder Kaliumcarbonat. Vorteilhaft wird eine wässrige Lösung von Natriumhydroxid oder Kaliumhydroxid verwendet, zum Beispiel mit einer Hydroxid-Konzentration der Lösung von 0,1 bis 10 molar, bevorzugt etwa 1 molar.

Die Aufarbeitung des Reaktionsgemisches kann nach jedem der beiden Verfahrensschritte a) und b) erfolgen. Die Synthese der Verbindungen der Formel Ia nach dem erfindungsgemäßen Verfahren kann aber auch in einer Eintopf-Reaktion ohne Isolierung des im Schritt a) gebildeten Thioharnstoffs der Formel IV erfolgen und eine Aufarbeitung nur nach dem Ablauf beider Verfahrensschritte durchgeführt werden. Die Aufarbeitung und gewünschtenfalls die Reinigung der Produkte erfolgt nach den üblichen Methoden wie Extraktion, Filtration, pH-Trennung, Chromatographie oder Kristallisation und den üblichen Trocknungen.

Die Ausgangsverbindungen der Formeln IIa und IIIa sind käuflich erhältlich oder können nach oder analog zu in der Literatur beschriebenen, dem Fachmann bekannten Verfahren hergestellt werden. In den Ausgangsverbindungen können auch funktionelle Gruppen in geschützter Form oder in Form von Vorstufen vorliegen und dann in den nach dem erfindungsgemäßen Verfahren hergestellten Verbindungen der Formel I in die gewünschten Gruppen überführt werden. Entsprechende Schutzgruppentechniken sind dem Fachmann bekannt. Beispielsweise kann in Verbindungen der Formel III, in denen X für NR5 steht, die Gruppe NHR5 in durch eine Acetyl-, Trifluoracetyl- oder Tritylgruppe geschützter Form vorliegen und vor der Durchführung von Verfahrensschritt b) entschützt werden.

X ist bevorzugt NH.

Der Rest Ar ist bevorzugt Phenyl oder ein monocyclischer Heteroaromat, besonders bevorzugt Phenyl oder ein fünfgliedriger Heteroaromat, zum Beispiel Thiophen oder Isoxazol, wobei alle diese Reste unsubstituiert oder substituiert sein können. Substituenten am aromatischen Rest Ar sind bevorzugt unabhängig voneinander ausgewählt aus der Gruppe (C1-C4)-Alkyl, F, Cl, Br und O(C1-C4)-Alkyl, wobei die Wasserstoffatome der Alkylreste ganz oder teilweise durch Fluoratome ersetzt sein können. Besonders bevorzugte Substituenten am Rest Ar sind unabhängig voneinander Methyl, Cl oder Br.

n, ist bevorzugt Null oder 1, besonders bevorzugt Null.

Bevorzugt stehen R1, R2, R3 und R4 unabhängig voneinander für Wasserstoff oder Methyl, besonders bevorzugt für Wasserstoff, oder R1 und R3 bilden zusammen eine Bindung und R2 und R4 bilden einen aromatischen Sechsring, bevorzugt einen Benzolring, wobei der aromatische Sechsring unsubstituiert oder mit 1, 2, 3 oder 4 voneinander unabhängigen Resten R7 substituiert sein kann, oder R1 und R3 stehen unabhängig voneinander für Wasserstoff oder Methyl und R2 und R4 bilden einen gesättigten 5- oder 6-gliedrigen Ring, bevorzugt einen Cyclopentan- oder Cyclohexanring, wobei der Ring mit 1, 2, 3, 4, 5 oder 6 voneinander unabhängigen Resten R8 substituiert sein kann.

R5 ist bevorzugt Wasserstoff oder Methyl, besonders bevorzugt Wasserstoff.

R7 ist bevorzugt unabhängig voneinander ausgewählt aus der Gruppe (C1-C4)-Alkyl, F, Cl, Br, OH oder O(C1-C4)-Alkyl, wobei die Wasserstoffatome der Alkylreste ganz oder teilweise durch Fluoratome ersetzt sein können; besonders bevorzugt sind die Substituenten R7 unabhängig voneinander F, Cl, Methyl, Methoxy, CF3 oder OH.

R8 ist bevorzugt unabhängig voneinander ausgewählt aus der Gruppe (C1-C4)-Alkyl oder O(C1-C4)-Alkyl, wobei die Wasserstoffatome der Alkylreste ganz oder teilweise durch Fluoratome ersetzt sein können.

Bei der Base handelt es sich bevorzugt um eine wässrige Base, Triethylamin oder Diisopropylethylamin, besonders bevorzugt um eine wässrige Metallhydroxid-Lösung, insbesondere um eine Natriumhydroxid- oder Kaliumhydroxidlösung.

Bei dem Sulfonsäurechlorid R6SO₂Cl handelt es sich um unsubstituierte oder substituierte Benzolsulfonsäurechloride, wobei R6 bevorzugt Phenyl oder p-Tolyl ist.

Die Verbindungen der Formel Ia können in Form ihrer Salze isoliert werden. Diese werden nach den üblichen Methoden durch Umsetzung mit Säuren oder Basen erhalten. Als Säureadditionssalze kommen dabei beispielsweise Halogenide, insbesondere Hydrochloride oder Hydrobromide, Lactate, Sulfate, Citrate, Tartrate, Acetate, Phosphate, Methylsulfonate, Benzolsulfonate, p-Toluolsulfonate, Adipinate, Fumarate, Gluconate, Glutamate, Glycerolphosphate, Maleinate, Benzoate, Oxalate und Pamoate und Trifluoracetate in Frage, im Fall der Herstellung von Wirkstoffen bevorzugt physiologisch akzeptable Salze. Enthalten die Verbindungen eine Säuregruppe, können sie Salze mit Basen bilden, beispielsweise Alkalimetallsalze, vorzugsweise Natrium- oder Kaliumsalze, oder Ammoniumsalze, zum Beispiel als Salze mit Ammoniak oder organischen Aminen oder Aminosäuren. Sie können auch als Zwitterion vorliegen.

Die Verbindungen der Formel Ia können weiterhin als Tautomere oder als Gemisch tautomerer Strukturen vorliegen.

Enthalten die Verbindungen der Formel Ia ein oder mehrere Asymmetriezentren, so können diese unabhängig voneinander sowohl S als auch R konfiguriert sein. Die Verbindungen können als optische Isomere, als Diastereomere, als cis/trans Isomere, als Racemate oder als Gemische derselben in beliebigen Verhältnissen vorliegen.

Wenn n = 0 ist, liegt eine direkte Bindung zwischen den beiden jeweils benachbarten Atomen vor.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl) und tert-Butyl (= 1,1-Dimethylethyl). Bevorzugte Alkylreste sind Methyl, Ethyl und Isopropyl. In Alkylresten können ein oder mehrere, zum Beispiel 1, 2, 3, 4, 5, 6, 7, 8 oder 9, Wasserstoffatome durch Fluoratome substituiert sein. Beispiele für solche Fluoralkylreste sind Trifluormethyl, 2,2,2-Trifluorethyl, Pentafluorethyl, Heptafluorisopropyl. Substituierte Alkylreste können in beliebigen Positionen substituiert sein, beispielsweise mit Fluor, mit Alkyl, zum Beispiel Methyl, Ethyl, Propyl, Butyl, oder mit Cycloalkyl, zum Beispiel Cyclopropyl, Cyclobutyl, Cyclopentyl oder Cyclohexyl.

Alkenylreste können geradkettig oder verzweigt sein. Dies gilt auch, wenn sie Substituenten tragen, beispielsweise in Fluoralkenylresten. Die Alkenylreste können in unterschiedlichen Positionen ungesättigt und auch mehrfach ungesättigt sein. Beispiele für Alkenylreste sind Ethenyl, n-Prop-1-enyl, n-Prop-2-enyl, Isoprop-1-enyl (= 1-Methylethenyl), n-But-1-enyl, n-But-2-enyl, n-But-3-enyl, n-Buta-1,3-dienyl, Isobut-1-enyl (= 2-Methylprop-1-enyl), Isobut-2-enyl (= 2-Methylprop-2-enyl), sec-But-1-enyl (= 1-Methylprop-1-enyl) und Pentenyl. Bevorzugte Alkenylreste sind Ethenyl, n-Prop-1-enyl, n-Prop-2-enyl, n-But-1-enyl, n-But-2-enyl, n-Pentenyl, n-Pentadienyl, Isopentenyl, tert-Pentenyl und Neopentenyl. In Alkenylresten können ein oder mehrere, zum Beispiel 1, 2, 3, 4, 5, 6, 7, 8 oder 9, Wasserstoffatome durch Fluoratome substituiert sein. Substituierte Alkenylreste können in beliebigen Positionen substituiert sein, beispielsweise mit Fluor, mit Alkyl, zum Beispiel Methyl, Ethyl, Propyl, Butyl, oder mit Cycloalkyl, zum Beispiel Cyclopropyl, Cyclobutyl, Cyclopentyl oder Cyclohexyl.

Alkinylreste können geradkettig oder verzweigt sein. Dies gilt auch, wenn sie Substituenten tragen, beispielsweise in Fluoralkinylresten. Die Alkinylreste können in unterschiedlichen Positionen ungesättigt und auch mehrfach ungesättigt sein. Beispiele für Alkinylreste sind Ethinyl, n-Prop-1-inyl, n-Prop-2-inyl, n-But-1-inyl, n-But-2-inyl, n-But-3-inyl, n-Buta-1,3-diinyl, sec-But-2-inyl (= 1-Methylprop-2-inyl), n-Pentinyl, n-Pentadiinyl, Isopentinyl, tert-Pentinyl und Neopentinyl. Bevorzugte Alkinylreste sind n-Prop-1-inyl, n-Prop-2-inyl, n-But-1-inyl und n-But-2-inyl. In Alkinylresten können ein oder mehrere, zum Beispiel 1, 2, 3, 4, 5, 6 oder 7, Wasserstoffatome durch Fluoratome substituiert sein. Substituierte Alkinylreste können in beliebigen Positionen substituiert sein, beispielsweise mit Fluor, mit Alkyl, zum Beispiel Methyl, Ethyl, Propyl, Butyl, oder mit Cycloalkyl, zum Beispiel Cyclopropyl, Cyclobutyl, Cyclopentyl oder Cyclohexyl. Beispiele für Cycloalkylreste sind Cyclopropyl, Cyclobutyl, Cyclopentyl, Cyclohexyl, Cycloheptyl oder Cyclooctyl. Bevorzugte Cycloalkylreste sind Cyclopropyl, Cyclopentyl und Cyclohexyl. In Cycloalkylresten können ein oder mehrere, zum Beispiel 1, 2, 3, 4, 5, 6, 7, 8, 9, 10, 11, 12, 13, 14 oder 15 Wasserstoffatome durch Fluoratome substituiert sein. Substituierte Cycloalkylreste können in beliebigen Positionen substituiert sein, beispielsweise mit Fluor, mit Alkyl, zum Beispiel Methyl, Ethyl, Propyl, Butyl, oder mit Cycloalkyl, zum Beispiel Cyclopropyl, Cyclobutyl, Cyclopentyl oder Cyclohexyl.

Die Cycloalkenylreste können in unterschiedlichen Positionen ungesättigt und auch mehrfach ungesättigt sein. Beispiele für Cycloalkenylreste sind Cyclobut-1-enyl, Cyclobut-2-enyl, Cyclopentenyl, Cyclopentadienyl, Cyclohexenyl, Cyclohexadienyl, Cycloheptenyl und Cyclooctenyl. Bevorzugte Cycloalkylenreste sind Cyclopentenyl, Cyclopentadienyl, Cyclohexenyl und Cyclohexadienyl. In Cycloalkenylresten können ein oder mehrere, zum Beispiel 1, 2, 3, 4, 5, 6, 7, 8, 9, 10, 11, 12 oder 13 Wasserstoffatome durch Fluoratome substituiert sein. Substituierte Cycloalkenylreste können in beliebigen Positionen substituiert sein, beispielsweise mit Fluor, mit Alkyl, zum Beispiel Methyl, Ethyl, Propyl, Butyl, oder mit Cycloalkyl, zum Beispiel Cyclopropyl, Cyclobutyl, Cyclopentyl oder Cyclohexyl.

Aromatische Ringsysteme sind Phenyl-, Naphthyl- und Heteroarylreste, und auch aromatische Kohlenstoff-Sechsringe, in denen ein oder zwei Kohlenstoffatome durch Stickstoff ersetzt sein können.

Phenylreste können unsubstituiert sein oder einfach oder mehrfach, zum Beispiel einfach, zweifach, dreifach, vierfach oder fünffach, durch gleiche oder verschiedene Reste substituiert sein. Wenn ein Phenylrest substituiert ist, trägt er bevorzugt einen oder zwei gleiche oder verschiedene Substituenten. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden. Naphthylreste können über alle Positionen angebunden sein, zum Beispiel über die 1-Position oder 2-Position. Naphthylreste können ebenfalls unsubstituiert sein oder einfach oder mehrfach, zum Beispiel einfach, zweifach, dreifach, vierfach oder fünffach, durch gleiche oder verschiedene Reste substituiert sein. Wenn ein Naphthylrest substituiert ist, trägt er bevorzugt einen oder zwei gleiche oder verschiedene Substituenten.

Heteroarylreste sind aromatische Ringverbindungen, in denen 1, 2, 3, oder 4 Ringatome Sauerstoffatome, Schwefelatome oder Stickstoffatome sind, z. B. 1, 2 oder 3 Stickstoffatome, 1 oder 2 Sauerstoffatome, 1 oder 2 Schwefelatome oder eine Kombinationen aus verschiedenen Heteroatomen. Die Heteroarylreste können mono - oder bicyclisch sein. Die Heteroarylreste können über alle Positionen gebunden sein, zum Beispiel über die 1-Position, 2-Position, 3-Position, 4-Position, 5-Position, 6-Position, 7-Position oder 8-Position. Heteroarylreste können unsubstituiert sein oder einfach oder mehrfach, zum Beispiel einfach, zweifach oder dreifach, durch gleiche oder verschiedene Reste substituiert sein.

Als Heteroarylreste kommen zum Beispiel in Frage:

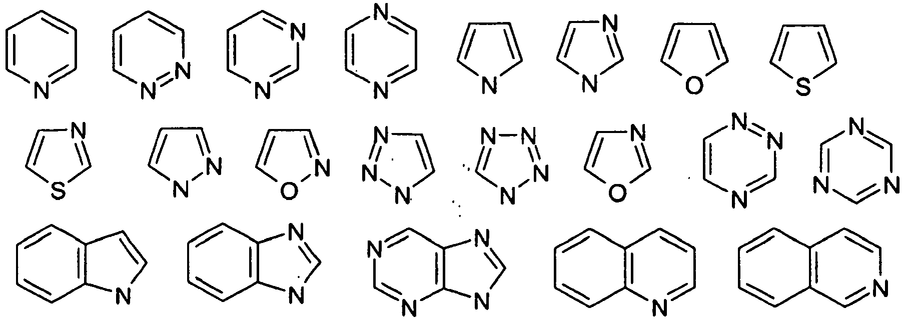

Bevorzugte Heteroarylreste sind monocyclische aromatische Ringverbindungen, besonders bevorzugt sind fünfgliedrige Heteroarylreste, zum Beispiel Thiophen und Isoxazol.

Wenn Gruppen, Substituenten oder Variable mehrfach in den Verbindungen der Formel Ia, IIa, IIIa, oder IVa vorkommen können, so können sie alle unabhängig voneinander die angegebenen Bedeutungen haben und können jeweils gleich oder verschieden sein.

In einer weiteren Ausführungsform ist Gegenstand der vorliegenden Erfindung ein Verfahren zur Herstellung von Verbindungen der Formel Ia, 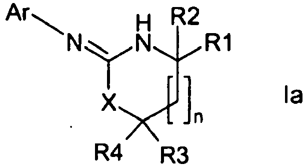 dadurch gekennzeichnet, dass
ein Thioharnstoff der Formel IVa mit einem Sulfonsäurechlorid R6SO₂Cl in Gegenwart einer Base in die Verbindung der Formel Ia überführt wird, 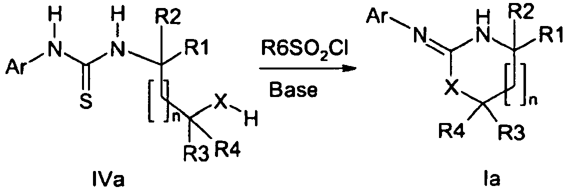 wobei
Ar, X, n, R1 bis R4 und R6 wie oben beschrieben definiert sind.
Alle Definitionen und Erläuterungen zu dem oben beschriebenen Verfahren gelten für dieses Verfahren entsprechend.

Die nach dem erfindungsgemäßen Verfahren erhältlichen Verbindungen der Formel Ia sind wertvolle Zwischenprodukte, zum Beispiel für die Herstellung von Arzneimittelwirkstoffen wie Clonidin und dessen Analoga, oder sind auch selbst Arzneimittelwirkstoffe. So werden zum Beispiel in den Anmeldungen WO03101984 und W003053434 Verbindungen beschrieben, die mittels des hier beschriebenen Verfahrens hergestellt werden können, und die sich als NHE Inhibitoren eignen, insbesondere NHE3 Inhibitoren, beispielsweise zur Behandlung von Atemstörungen und des Schnarchens sowie zur Verbesserung des Atemantriebes, oder zur Behandlung von akuten oder chronischen Erkrankungen, die durch ischämische und/oder Reperfusionsereignisse oder durch proliferative oder durch fibrotische Ereignisse ausgelöst werden.

### Versuchsbeschreibungen und Beispiele:

Abkürzungen:
- abs.: absolut
- ESI: Elektrospray-Ionisation
- rt: Retentionszeit
- THF: Tetrahydrofuran
- TFA: Trifluoressigsäure

Die im Folgenden angegebenen Retentionszeiten (rt) beziehen sich auf LCMS-Messungen mit den folgenden Parametern:
Analytische Methoden:
Methode A:

| | |
|---|---|
| stationäre Phase: | Merck Purospher 5µ 2 x 55 mm |
| mobile Phase: | 95% H₂O (0,05% TFA)→ 95% Acetonitril, 3 min; → 95% Acetonitril, 1,5 min; 0,5 ml/min. |

Methode B:

| | |
|---|---|
| stationäre Phase: | Merck Purospher 3µ 2 x 55 mm |
| mobile Phase: | 95% H₂O (0,08% HCOOH) → 95% Acetonitril (0,1 % HCOOH), 5 min; → 95% Acetonitril (0,1% HCOOH), 2 min; → 95% H₂O (0,1% HCOOH), 1 min; 0,45 ml/min. |

Methode C:

| | |
|---|---|
| stationäre Phase: | YMC J'sphere H80, 4µ, 2,1x20 mm |
| mobile Phase: | 96% H₂O (0,05% TFA)→ 95% Acetonitril, 2 min; → 95% Acetonitril, 0,4 min; 1 ml/min. |

Methode D:

| | |
|---|---|
| stationäre Phase: | YMC J'sphere H80, 4µ, 2,1x20 mm |
| mobile Phase: | 95% H₂O (0,05% TFA)→ 95% Acetonitril, 2,3 min; → 95% Acetonitril, 1 min; 1 ml/min. |

Die präparative HPLC wurde unter folgenden Bedingungen durchgeführt:

| | |
|---|---|
| stationäre Phase: | Merck Purospher RP18 (10µm) 250 x 25 mm |
| mobile Phase: | 90% H₂O (0,05% TFA)→ 90% Acetonitril, 40 min; 25 ml/min |

### Beispiel 1: Imidazolidin-2-yliden-phenyl-amin, Trifluoressigsäuresalz

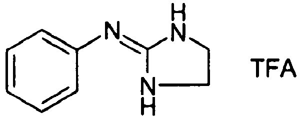

### a) 1-(2-Amino-ethyl)-3-phenyl-thiohamstoff

Zu einer Lösung aus Ethylendiamin (5,56 g ) in abs. THF (6 ml) wurde unter Argon eine Lösung aus Phenylisothiocyanat (500 mg) in abs. THF (6 ml) tropfenweise über 20 Minuten zugegeben. Danach wurde das Reaktionsgemisch auf Wasser gegeben, mit 10 %iger HCl angesäuert und mit Essigester extrahiert. Dann wurde die wässrige Phase mit Kaliumcarbonat basisch gemacht und drei Mal mit Essigester extrahiert. Die vereinigten organischen Phasen wurden über Magnesiumsulfat getrocknet, filtriert und eingeengt. Anschließend wurde zwei Mal mit Toluol koevaporiert. Zurück blieben 650 mg des gewünschten Produkts.
LCMS-rt (A): 1,96 min
MS (ESI+): 196,2

### b) Imidazolidin-2-yliden-phenyl-amin, Trifluoressigsäuresalz

1-(2-Amino-ethyl)-3-phenyl-thioharnstoff (50 mg) wurde unter Argon in THF (1,5 ml) gelöst, mit einer Lösung aus Natriumhydroxid (25,6 mg) in Wasser (0,6 ml) versetzt und innerhalb von fünf Minuten eine Lösung aus p-Toluolsulfonsäurechlorid (53,7 mg) in THF zugetropft. Nach einer halben Stunde Rühren wurde das Reaktionsgemisch auf Wasser gegeben und sechs Mal mit Ether extrahiert. Anschließend wurden die vereinigten organischen Phasen über Magnesiumsulfat getrocknet, filtriert und eingeengt. Der Rückstand wurde mittels präparativer Chromatographie gereinigt, die Produkt-enthaltenden Fraktionen vereinigt, vom Acetonitril befreit und gefriergetrocknet. Nach Gefriertrocknung wurden 20 mg des gewünschten Produkts erhalten.
LCMS-rt (A): 1,72 min
MS (ESI+): 162,2

### Beispiel 2: [1,3]Oxazinan-2-yliden-phenyl-amin

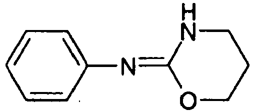

### a) 1-(3-Hydroxy-propyl)-3-phenyl-thioharnstoff

Zu einer Lösung aus 3-Amino-1-propanol (114,5 mg) in abs. THF (2 ml) wurde unter Argon und Rühren eine Lösung aus Phenylisothiocyanat (200 mg) in abs. THF (2 ml) zugetropft. Das Reaktionsgemisch wurde zwei Stunden bei Raumtemperatur gerührt. Nach Abziehen des Lösungsmittels wurde der Rückstand in wässriger HCl gelöst und mit Ether gewaschen. Anschließend wurde die wässrige Phase mit Kaliumcarbonat basisch gemacht und drei Mal mit Ether extrahiert. Die vereinigten organischen Phasen wurden über Magnesiumsulfat getrocknet, filtriert und eingeengt. Der Rückstand wurde mittels präparativer Chromatographie gereinigt, die Produkt-enthaltenden Fraktionen vereinigt, vom Acetonitril befreit, basisch gestellt und drei Mal mit Essigester extrahiert. Die organischen Phasen wurden vereinigt, getrocknet (MgSO₄) und filtriert. Nach Abziehen des Lösungsmittels wurden 114 mg des gewünschten Produkts erhalten.
LCMS-rt (B): 1,99 min
MS (ESI+): 211,20

### b) [1,3]Oxazinan-2-yliden-phenyl-amin

Zu einer Lösung aus 1-(3-Hydroxy-propyl)-3-phenyl-thioharnstoff (50 mg) und THF (1,5 ml) wurde unter Argon und Rühren eine Lösung aus Natriumhydroxid (23,8 mg) und Wasser (0,6 ml) gegeben. Anschließend wurde über fünfzehn Minuten eine Lösung aus p-Toluolsulfonsäurechlorid (49,9 mg) und THF (0,5 ml) zugetropft. Nach 30 Minuten Rühren wurde das Reaktionsgemisch auf Wasser gegeben und drei Mal mit Ether extrahiert. Die vereinigten organischen Phasen wurden über Magnesiumsulfat getrocknet, filtriert und eingeengt. Chromatographie über Kieselgel (anfangs Methylenchlorid/Methanol 50:1, am Ende Methanol/gesättigte Ammoniak-Lösung 100:1) lieferte 27,4 mg des gewünschten Produkts.
NMR (400 MHz, CDCl3): 7,35-7,18 (4H, m), 6,9-7,0 (1 H, m), 4,29 (2H, t), 3,43 (2H, t), 1,96 (2H, q)

### Beispiel 3: (2,6-Dichlor-phenyl)-(octahydro-benzoimidazol-2-yliden)-amin

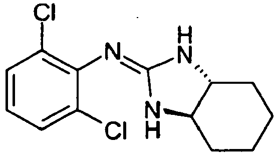

a) 1-(2-Amino-cyclohexyl)-3-(2,6-dichlor-phenyl)-thioharnstoff Zu einer Lösung aus trans-1,2-Diaminocyclohexan (139,9 mg) und abs. THF (3 ml) wurde über eine halbe Stunde langsam eine Lösung aus 1,3-Dichlor-2-isothiocyanato-benzol (100 mg) und abs. THF (3 ml) getropft. Die Lösung wurde noch 90 Minuten bei Raumtemperatur gerührt. Anschließend wurde das Reaktionsgemisch auf Wasser gegeben, mit Salzsäure angesäuert und einmal mit Ethylacetat extrahiert. Danach wurde mit Kaliumcarbonat basisch gemacht und dreimal mit Ethylacetat extrahiert. Die vereinigten organischen Phasen wurden über Magnesiumsulfat getrocknet, filtriert und eingeengt. Es wurden 128 mg des gewünschten Produkts erhalten.
   LCMS-rt (B): 1,88 min
   MS (ESI+): 318,20
b) : (2,6-Dichlor-phenyl)-(octahydro-benzoimidazol-2-yliden)-amin Zu einer Lösung aus 1-(2-Amino-cyclohexyl)-3-(2,6-dichlor-phenyl)-thioharnstoff (50 mg) und THF (1,5 ml) wurde unter Argon eine Lösung aus Natriumhydroxid (15,7 mg) und Wasser (0,6 ml) gegeben. Anschließend wurde über fünfzehn Minuten eine Lösung aus p-Toluolsulfonsäurechlorid (32,9 mg) und THF (0,5 ml) zugetropft. Nach 60 Minuten Rühren wurde das Reaktionsgemisch auf Wasser gegeben und drei Mal mit Ether extrahiert. Die vereinigten organischen Phasen wurden über Magnesiumsulfat getrocknet, filtriert und eingeengt. Es wurden 44 mg des gewünschten Produkts erhalten.
   LCMS-rt (B): 1,95 min
   MS (ESI+): 284,20

### Beispiel 4: (5-Fluor-1 H-benzoimidazol-2-yl)-(4-methyl-thiophen-3-yl)-amin Hydrochlorid

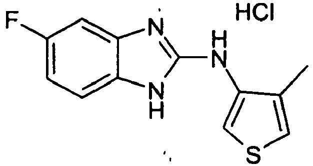

a) 1-(2-Amino-5-fluor-phenyl)-3-(4-methyl-thiophen-3-yl)-thioharnstoff und 1-(2-Amino-4-fluor-phenyl)-3-(4-methyl-thiophen-3-yl)-thioharnstoff
   4-Fluor-o-phenylen-diamin (1,5 g) wurde in abs. THF (25 ml) gelöst und unter Rühren 3-Isothiocyanato-4-methyl-thiophen (1,8 g), gelöst in abs. THF (25 ml) zugetropft. Nach beendeter Zugabe wurde 3 h bei Raumtemperatur gerührt, dann wurde noch etwas 3-Isothiocyanato-4-methyl-thiophen zugegeben und eine weitere Stunde gerührt. Nach Stehenlassen über Nacht wurde das THF abgezogen, der Rückstand in Ethanol gelöst, Kohle zugesetzt, zum Sieden erhitzt und heiß filtriert. Aus dem Filtrat wurde nach Abkühlen 1,8 g des gewünschten Produkts mit Ether ausgefällt.
b) (5-Fluor-1H-benzoimidazol-2-yl)-(4-methyl-thiophen-3-yl)-amin Hydrochlorid
   Das Gemisch aus 1-(2-Amino-5-fluor-phenyl)-3-(4-methyl-thiophen-3-yl)-thioharnstoff und 1-(2-Amino-4-fluor-phenyl)-3-(4-methyl-thiophen-3-yl)-thioharnstoff (1,75 g) wurde in THF (50 ml) gelöst und mit einer Lösung aus Natriumhydroxid (0,622 g) und Wasser (15 ml) versetzt. Innerhalb von 5 min. wurde eine Lösung aus p-Toluolsulfonäurechlorid (1,304 g) und THF (10 ml) zugetropft. Nach beendeter Zugabe wurde eine halbe Stunde bei Raumtemperatur gerührt. Das Reaktionsgemisch wurde auf Wasser gegossen und die Wasserphase drei Mal mit Ether extrahiert. Die vereinigten Etherphasen wurden mit Magnesiumsulfat getrocknet, filtriert und eingeengt. Das Rohprodukt wurde in Essigester gelöst und mit ätherischer HCl auf pH 2 gestellt. Durch Zugabe von Ether wurde das Produkt ausgefällt. Nach Trocknen wurden 750 mg des gewünschten Produkts erhalten.
   LCMS-rt (B): 1,48 min
   MS (ESI+): 248,11

Ausgehend von käuflichen oder bekannten Ausgangsmaterialien wurden folgende Verbindungen analog zu obigen Beispielen hergestellt:

| Beispiel | Amin | Isothiocyanat | Produkt | Fp [°C] | LCMS rt [min] | MS (ESI⁺, M+H⁺) |
|---|---|---|---|---|---|---|
| 5* | 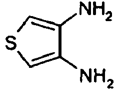 | 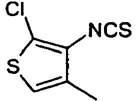 | 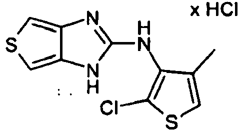 | >300 | | |
| 6* | 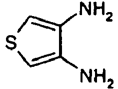 | 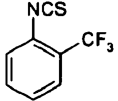 | 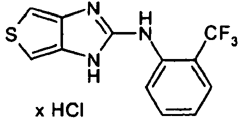 | 194-196 | | |
| 7* | 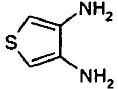 | 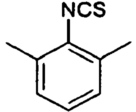 | 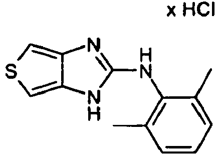 | >310 | | |
| 8* | 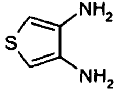 | 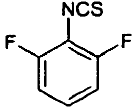 | 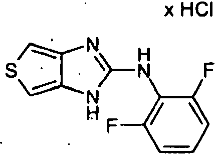 | 296 | | |
| 9* | 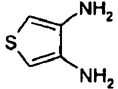 | 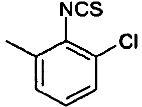 | 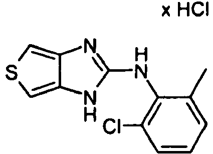 | >310 | | |
| 10* | 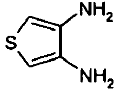 | 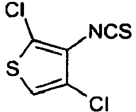 | 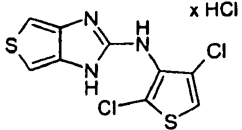 | >300 | | |
| 11 | 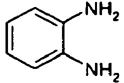 | 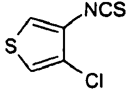 | 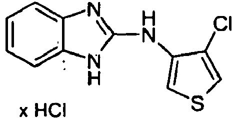 | 256-260 | | |
| 12 | 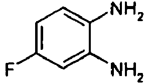 | 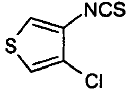 | 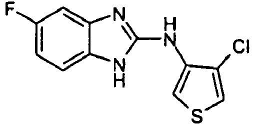 | | 0,90 (C) | 268,0 |
| 13 | 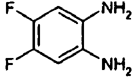 | 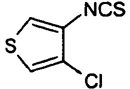 | 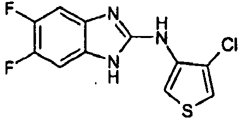 | | 0,95 (C) | 286,0 |
| 14 | 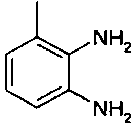 | 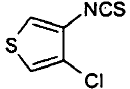 | 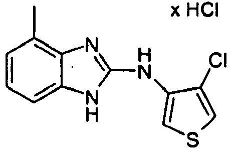 | 325-327 | | |
| 15 | 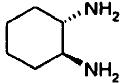 | 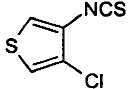 | 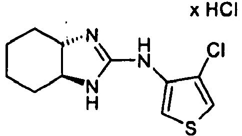 | 196-200 | | |
| 16 | 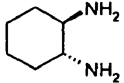 | 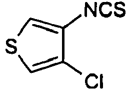 | 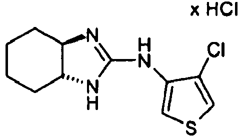 | 240-244 | | |
| 17 | 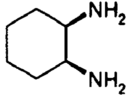 | 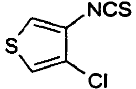 | 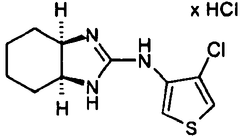 | 228-231 | | |
| 18 | 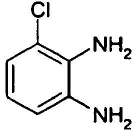 | 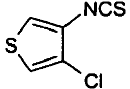 | 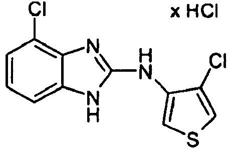 | 276-280 | | |
| 19 | 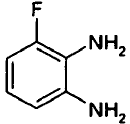 | 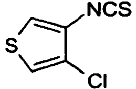 | 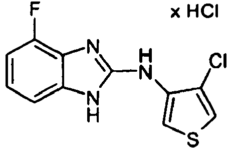 | | 0,89 (C) | 268,0 |
| 20 | 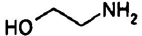 | 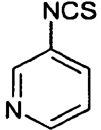 | 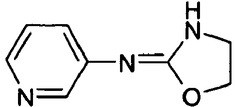 | | 0,14 (C) | 164,1 |
| 21 | 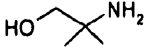 | 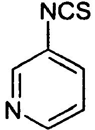 | 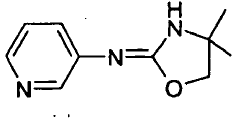 | | 0,20 (C) | 192,1 |
| 22 | 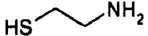 | 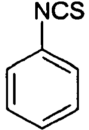 | 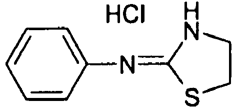 | | 0,64 (C) | 179,1 |
| 23 | 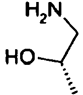 | 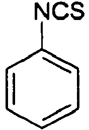 | 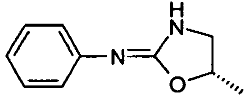 | | 0,71 (C) | 177,1 |
| 24 | 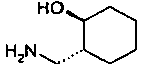 | 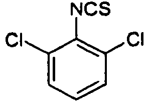 | 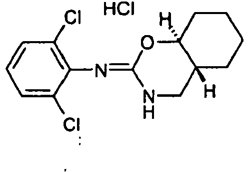 | | 1,07 (C) | 299,4 |
| 25 | 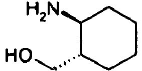 | 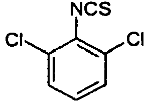 | 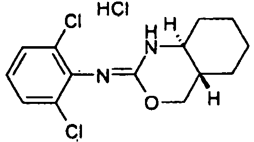 | | 1,04 (C) | 299,3 |
| 26 | 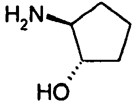 | 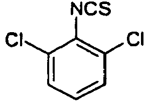 | 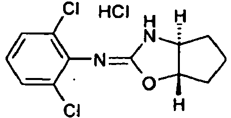 | | 1,83 (D) | 271,3 |
| 27 | 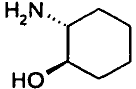 | 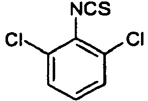 | 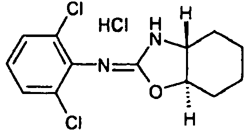 | | 1,83 (D) | 285,3 |
| 28 | 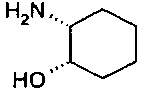 | 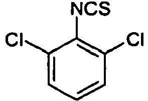 | 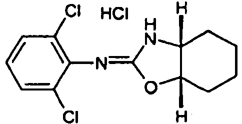 | | 1,76 (D) | 285,3 |

*Die Beispielverbindungen 5 bis 10 sind nicht erfindungsgemäß beansprucht.

### Beispiel 29 : (2,6-Dichlorphenyl)-imidazolidin-2-yliden-amin

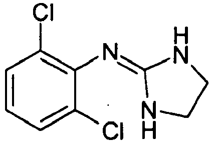

a) 1-(2-Amino-ethyl)-3-(2,6-dichlorphenyl)-thioharnstoff
   Zu einer Lösung aus Ethylendiamin (3,68 g) und abs. THF (4 ml) wurde unter Argon eine Lösung aus 2,6-Dichlorphenylisothiocyanat (500 mg) und THF (5 ml) in 20 Minuten getropft. Nach weiteren 30 min Rühren wurde das Gemisch auf Wasser gegeben, mit 10 %-iger HCl angesäuert und dreimal mit Essigester extrahiert. Die wässrige Phase wurde mit gesättigter Kaliumcarbonat-Lösung basisch gestellt und dreimal mit EE extrahiert. Die vereinigten organischen Phasen wurden über Magnesiumsulfat getrocknet, das Lösungsmittel im Vakuum entfernt und der Rückstand zweimal mit Toluol koevaporiert. Nach Trocknen im Hochvakuum wurde das gewünschte Produkt als weißer Feststoff erhalten (532 mg).
   LCMS-rt (C): 0,719 min
   MS (ESI+): 264,0
b) (2,6-Dichlorphenyl)-imidazolidin-2-yliden-amin
   1-(2-Amino-ethyl)-3-(2,6-dichlorphenyl)-thioharnstoff (200 mg) wurde unter Argon in THF (4 ml) gelöst, mit einer Lösung aus Natriumhydroxid (102 mg) in Wasser (2 ml) versetzt und dann in fünf Minuten eine Aufschlämmung aus polystyrolgebundenem Toluolsulfonsäurechlorid (457 mg, 2,9 mmol/g) in THF (4 ml) zugetropft. Nach 2 h Rühren bei Raumtemperatur wurden weiteres polystyrolgebundenes Toluolsulfonsäurechlorid (65 mg in 2 ml THF) zugegeben, nach einer weiteren Stunde gefolgt von weiterem Säurechlorid (124 mg in 2 ml THF). Nach Stehen über Nacht wurde der Reaktionsansatz filtriert, das Harz zweimal in Dichlormethan aufgeschlämmt und die vereinigten Phasen zur Trockne eingeengt. Der Rückstand wurde in Wasser/Dichlormethan aufgenommen, die Phasen getrennt und die wässrige Phase dreimal mit Dichlormethan extrahiert. Die vereinigten organischen Phasen wurden über Magnesiumsulfat getrocknet, das Lösungsmittel im Vakuum entfernt und anschließend im Hochvakuum getrocknet. Es wurden 104 mg der Titelverbindung erhalten.
   LCMS-rt (C): 0,65 min
   MS (ESI+): 230,1

Analog zu Beispiel 29 wurden folgende Verbindungen erhalten:

| Beispiel | Amin | Isothiocyanat | Produkt | LCMS rt [min] | MS (ES⁺, M+H⁺) |
|---|---|---|---|---|---|
| 30 | 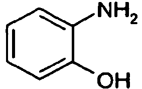 | 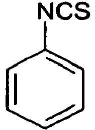 | 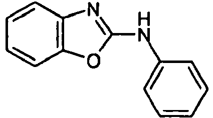 | 1,42 (C) | 211,1 |
| 31* | 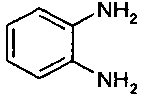 | 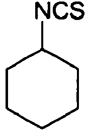 | 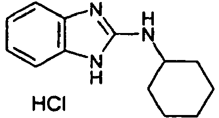 | 0,95 (C) | 216,1 |

| | | | | | |
|---|---|---|---|---|---|
| *Die Beispielverbindung 31 ist nicht erfindungsgemäß beansprucht. | | | | | |

## Patentansprüche

1. Verfahren zur Herstellung von Heterocyclen der Formel Ia, 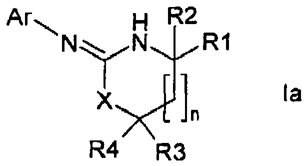 worin bedeuten:
X NR5, wobei R5 Wasserstoff oder (C1-C4)-Alkyl entspricht;
n Null, 1, 2 oder 3;
Ar Phenyl, Naphthyl oder Heteroaryl, die mit 1, 2, 3, 4 oder 5 Resten R11 substituiert sein können,
wobei R11 unabhängig voneinander aus der Gruppe (C1-C4)-Alkyl, F, Cl, Br, I, CN, NO₂, OH, O(C1-C4)-Alkyl, COO(C1-C4)-Alkyl gewählt ist und wobei die Wasserstoffatome der Alkylreste ganz oder teilweise durch Fluoratome ersetzt sein können;
R1, R2, R3 und R4 unabhängig voneinander Wasserstoff, F oder (C1-C4)-Alkyl, wobei die Wasserstoffatome der Alkylreste ganz oder teilweise durch Fluoratome ersetzt sein können;
oder
R1 und R3 zusammen eine Bindung,
und
R2 und R4 mit den beiden Kohlenstoffatomen, an die sie gebunden sind, einen aromatischen Kohlenstoff-Sechsring, in dem ein oder zwei Kohlenstoffatome durch Stickstoff ersetzt sein können, wobei der aromatische Sechsring mit 1, 2, 3 oder 4 Resten R7 substituiert sein kann,
wobei R7 unabhängig voneinander aus der Gruppe (C1-C4)-Alkyl, F, Cl, Br, I, CN, NO₂, OH, O(C1-C4)-Alkyl, COO(C1-C4)-Alkyl gewählt ist und wobei die Wasserstoffatome der Alkylreste ganz oder teilweise durch Fluoratome ersetzt sein können,
wobei n = 0 ist;
oder
R1 und R3 unabhängig voneinander Wasserstoff oder (C1-C4)-Alkyl
und
R2 und R4 mit den beiden Kohlenstoffatomen, an die sie gebunden sind, einen gesättigten 5-, 6-, 7- oder 8-gliedrigen Kohlenstoffring, in dem ein oder zwei Kohlenstoffatome durch O, S, NH und N(C1-C4)-Alkyl ersetzt sein können und der mit 1, 2, 3, 4, 5 oder 6 Resten R8 substituiert sein kann,
wobei R8 unabhängig voneinander aus der Gruppe (C1-C4)-Alkyl, O(C1-C4)-Alkyl, COO(C1-C4)-Alkyl gewählt ist, wobei die Wasserstoffatome der Alkylreste ganz oder teilweise durch Fluoratome ersetzt sein können,
wobei n = 0 ist;
und deren Tautomere und deren Salze,
**dadurch gekennzeichnet, dass**, wie in Schema 2 dargestellt, 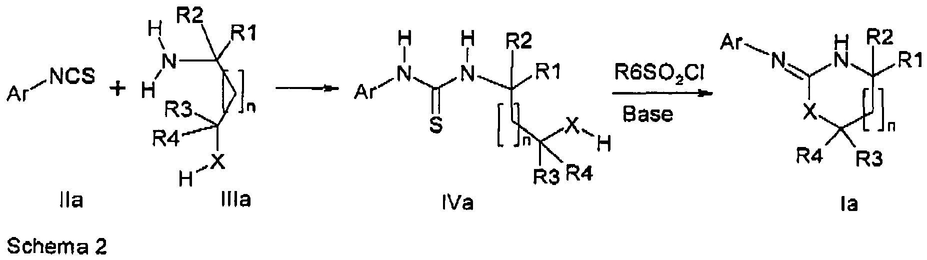
a) ein aromatisches Isothiocyanat der Formel IIa mit einem primären Amin der Formel IIIa zum Thioharnstoff der Formel IVa umgesetzt wird, und
b) der Thioharnstoff der Formel IVa mit einem Sulfonsäurechlorid R6SO₂Cl in Gegenwart einer Base in die Verbindung der Formel Ia überführt wird,
wobei in den Verbindungen der Formeln IIa, IIIa und IVa
Ar, X, n und R1 bis R4 definiert sind wie in Formel Ia und
R6 Phenyl ist, welches unsubstituiert ist oder substituiert ist mit Methyl, Trifluormethyl, F, Cl oder Br.

2. Verfahren nach Anspruch 1, bei dem die Reaktion als Eintopfreaktion durchgeführt wird.

3. Verfahren gemäß Anspruch 1 und/oder 2, wobei die Schritte a) und b) unabhängig voneinander kontinuierlich oder diskontinuierlich geführt werden.

4. Verfahren gemäß einem oder mehreren der Ansprüche 1 bis 3, worin Ar Phenyl, Thienyl oder Isoxazolyl ist, die wie in Anspruch 1 angegeben substituiert sein können.

5. Verfahren gemäß einem oder mehreren der Ansprüche 1 bis 4, worin R6 Phenyl oder p-Methylphenyl ist.

6. Verfahren gemäß einem oder mehrerer der Ansprüche 1 bis 5, worin die im Schritt b) eingesetzte Base Natriumhydroxid oder Kaliumhydroxid ist.

7. Verfahren zur Herstellung einer Verbindung der Formel Ia definiert wie in Anspruch 1, 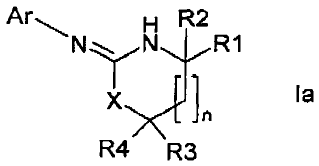 **dadurch gekennzeichnet, dass** ein Thioharnstoff der Formel IVa mit einem Sulfonsäurechlorid R6SO₂Cl in Gegenwart einer Base in die Verbindung der Formel Ia überführt wird, 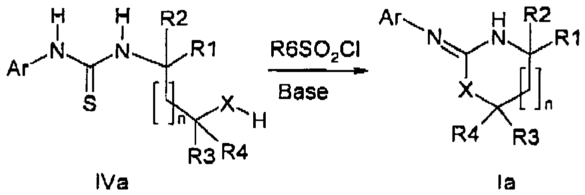 wobei
Ar, X, n, R1 bis R4 und R6 wie in Anspruch 1 definiert sind.

## Claims

1. A process for preparing heterocycles of the formula I 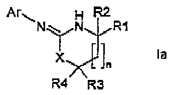 where:
X is NR5,
where R5 is hydrogen or (C1-C4)-alkyl;
n is zero, 1, 2 or 3;
Ar is phenyl, naphthyl or heteroaryl which may be substituted by 1, 2, 3, 4 or 5 R11 radicals
where R11 is in each case independently selected from the group of (C1-C4)-alkyl, F, Cl, Br, I, CN, NO₂, OH, O(C1-C4)-alkyl, COO(C1-C4)-alkyl, and some or all of the hydrogen atoms of the alkyl radicals may be replaced by fluorine atoms;
R1, R2, R3 and R4 are each independently hydrogen, F or (C1-C4)-alkyl where some or all of the hydrogen atoms of the alkyl radicals may be replaced by fluorine atoms;
or
R1 and R3 together are a bond, and
R2 and R4 with the two carbon atoms to which they are bonded are an aromatic six-membered carbocycle in which one or two carbon atoms may be replaced by nitrogen and the aromatic six-membered ring may be substituted by 1, 2, 3 or 4 R7 radicals,
where R7 is in each case independently selected from the group of (C1-C4)-alkyl, F, Cl, Br, I, CN, NO₂, OH, O(C1-C4)-alkyl, COO(C1-C4)-alkyl, and some or all of the hydrogen atoms of the alkyl radicals may be replaced by fluorine atoms,
where n = 0;
or
R1 and R3 are each independently hydrogen or (C1-C4)-alkyl
and
R2 and R4 with the two carbon atoms to which they are bonded are a saturated 5-, 6-, 7- or 8-membered carbocycle in which one or two carbon atoms may be replaced by O, S, NH and N(C1-C4)-alkyl and may be substituted by 1, 2, 3, 4, 5 or 6 R8 radicals
where R8 is in each case independently selected from the group of (C1-C4)-alkyl, O(C1-C4)-alkyl, COO(C1-C4)-alkyl, and some or all of the hydrogen atoms of the alkyl radicals may be replaced by fluorine atoms,
where n = 0;
and their tautomers and their salts,
which comprises, as shown in scheme 2 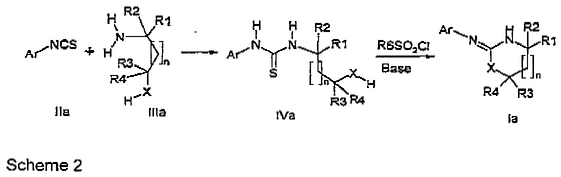
a) reacting an aromatic isothiocyanate of the formula IIa with a primary amine of the formula IIIa to give thiourea of the formula IVa, and
b) converting the thiourea of the formula IVa using a sulfonyl chloride R6SO₂Cl in the presence of a base to the compound of the formula la,
where, in the compounds of the formulae IIa, IIIa and IVa,
Ar, X, n and R1 to R4 are each as defined in formula Ia and
R6 is phenyl which is unsubstituted or substituted by methyl, trifluoromethyl, F, Cl or Br.

2. The process of claim 1, in which the reaction is carried out as a one-pot reaction.

3. The process of claim 1 and/or 2, wherein steps a) and b) are each independently conducted continuously or batchwise.

4. The process of one or more of claims 1 to 3, wherein Ar is phenyl, thienyl or isoxazolyl, each of which may be substituted as specified in claim 1.

5. The process of one or more of claims 1 to 4, wherein R6 is phenyl or p-methylphenyl.

6. The process of one or more of claims 1 to 5, wherein the base used in step b) is sodium hydroxide or potassium hydroxide.

7. A process for preparing a compound of the formula la as defined in claim 1 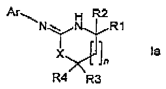 which comprises
converting a thiourea of the formula IVa using a sulfonyl chloride R6SO₂Cl in the presence of a base to the compound of the formula I 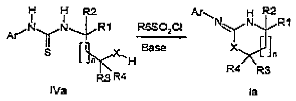 where
Ar, X, n, R1 to R4 and R6 are each as defined in claim 1.

## Revendications

1. Procédé pour la préparation d'hétérocycles de formule la, 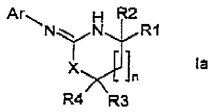 où :
X signifie NR5,
où R5 représente hydrogène ou (C1-C4)-alkyle ;
n vaut zéro, 1, 2 ou 3 ;
Ar signifie phényle, naphtyle ou hétéroaryle, qui peuvent être substitués par 1, 2, 3, 4 ou 5 radicaux R11,
R11 étant choisi, indépendamment les uns des autres, dans le groupe (C1-C4)-alkyle, F, CI, Br, I, CN, NO₂, OH, O(C1-C4)-alkyle, COO(C1-C4)-alkyle et les atomes d'hydrogène des radicaux alkyle pouvant être remplacés totalement ou partiellement par des atomes de fluor ;
R1, R2, R3 et R4 indépendamment les uns des autres, signifient hydrogène, F ou (C1-C4)-alkyle,
les atomes d'hydrogène des radicaux alkyle pouvant être remplacés totalement ou partiellement par des atomes de fluor ; ou
R1 et R3 signifient ensemble une liaison, et
R2 et R4 signifient, avec les deux atomes de carbone auxquels ils sont liés un noyau hexagonal carboné aromatique, dans lequel un ou deux atomes de carbone peuvent être remplacés par de l'azote, le noyau hexagonal aromatique pouvant être substitué par 1, 2, 3 ou 4 radicaux R7,
R7 étant choisi, indépendamment les uns des autres, dans le groupe (C1-C4)-alkyle, F, CI, Br, I, CN, NO₂, OH, O(C1-C4)-alkyle, COO(C1-C4)-alkyle et les atomes d'hydrogène des radicaux alkyle pouvant être remplacés totalement ou partiellement par des atomes de fluor,
où n = 0 ; ou
R1 et R3 signifient, indépendamment l'un de l'autre, hydrogène ou (C1-C4)-alkyle et
R2 et R4 signifient, avec les deux atomes de carbone auxquels ils sont liés, un noyau carboné saturé à 5, 6, 7 ou 8 chaînons, dans lequel un ou deux atomes de carbone peuvent être remplacés par O, S, NH et N(C1-C4)-alkyle et qui peut être substitué par 1, 2, 3, 4, 5 ou 6 radicaux R8,
R8 étant choisi, indépendamment les uns des autres, dans le groupe (C1-C4)-alkyle, O(C1-C4)-alkyle, COO(C1-C4)-alkyle, les atomes d'hydrogène des radicaux alkyle pouvant être remplacés totalement ou partiellement par des atomes de fluor,
où n = 0 ;
et leurs tautomères et leurs sels,
**caractérisé en ce que**, comme indiqué dans le schéma 2, 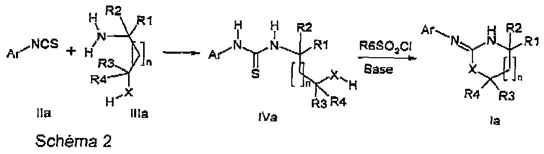
a) un isothiocyanate aromatique de formule IIa est transformé avec une amine primaire de formule IIIa en thio-urée de formule IVa, et
b) la thio-urée de formule IVa est transformée avec un chlorure d'acide sulfonique R6SO₂Cl en présence d'une base en composé de formule la,
où, dans les composés des formules IIa, IIIa et IVa
Ar, X, n et R1 à R4 sont définis comme dans la formule Ia et
R6 représente phényle, qui est non substitué ou substitué par méthyle, trifluorométhyle, F, Cl ou Ar.

2. Procédé selon la revendication 1, dans lequel la réaction est réalisée en tant que réaction dans un pot.

3. Procédé selon la revendication 1 et/ou 2, les étapes a) et b) étant réalisées, indépendamment l'une de l'autre, de manière continue ou discontinue.

4. Procédé selon l'une ou plusieurs des revendications 1 à 3, dans lequel Ar représente phényle, thiényle ou isoxazolyle, qui peuvent être substitués comme indiqué dans la revendication 1.

5. Procédé selon l'une ou plusieurs des revendications 1 à 4, dans lequel R6 représente phényle ou p-méthylphényle.

6. Procédé selon l'une ou plusieurs des revendications 1 à 5, dans lequel la base utilisée dans l'étape b) est l'hydroxyde de sodium ou l'hydroxyde de potassium.

7. Procédé pour la préparation d'un composé de formule la défini comme dans la revendication 1 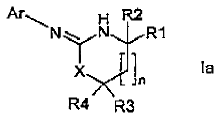 **caractérisé en ce qu'**une thio-urée de formule IVa est transformée avec un chlorure d'acide sulfonique R6SO₂Cl en présence d'une base en composé de formule Ia, 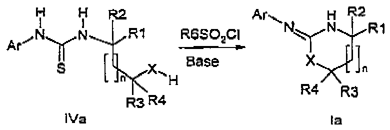 où
Ar, X, n, R1 à R4 et R6 sont définis comme dans la revendication 1.
